# EUROPEAN PATENT SPECIFICATION

(11) **EP 3 673 882 B1**
(45) Date of publication and mention of the grant of the patent: **09.11.2022**
(21) Application number: 19217845.7
(22) Date of filing: 19.12.2019
(51) Int. Cl.: A61F 13/496

(54) **PANTS-TYPE ABSORBENT ARTICLE**
HOSENÄHNLICHER SAUGFÄHIGER ARTIKEL
ARTICLE ABSORBANT DE TYPE CULOTTE

(30) Priority: 25.12.2018 JP 2018241693
(43) Date of publication of application: 01.07.2020
(73) Proprietor: Unicharm Corporation, Shikokuchuo-shi, Ehime 799-0111 (JP)
(72) Inventor: Kawabata, Kuniyoshi, Kanonji-shi, Kagawa 769-1602 (JP); Ichikawa, Makoto, Kanonji-shi, Kagawa 769-1602 (JP)
(74) Representative: Dolleymores

(56) References cited:
- EP-A1- 3 138 545
- JP-A- 2010 220 782
- JP-A- 2013 233 382
- US-A1- 2008 140 038

## Description

### FIELD

The present disclosure relates to a pants-type absorbent article.

### BACKGROUND

A pants-type disposable diaper is known, which comprises an abdominal-side part and a back-side part in which end parts of the abdominal-side part in a width direction and end parts of the back-side part in the width direction, which are superimposed so that the inner surfaces thereof face each other, are joined to each other by seal parts (refer to, for example, Patent Literature 1). The seal parts of this diaper each comprise a plurality of patterned fused parts which are spaced from each other in a length direction.

### [CITATION LIST]

### [PATENT LITERATURE]

[PTL 1] Japanese Unexamined Patent Publication (Kokai) No. 2004-330622

JP 2013233382 A discloses a pants-type absorbent article having a ventral side and a dorsal side and formed by joining both side edges of the ventral side and the dorsal side with a pair of side seal portions which have a plurality of seal patterns formed intermittently in the longitudinal direction of the article.

### SUMMARY

### [TECHNICAL PROBLEM]

In order to remove a pants-type diaper from the body of the wearer, the wearer or a caregiver may separate the abdominal-side part and back-side part from each other by tearing the seal parts of the pants-type diaper. In this case, as long as the seal parts are continuously torn in substantially the length direction, i.e., if the tear lines extend continuously in substantially the length direction, the separation of the abdominal-side part and the back-side part is completed quickly. Conversely, if the tear lines extend in substantially the width direction, the separation of the abdominal-side part and the back-side part is interrupted.

Patent Literature 1 neither discloses nor suggests such a technical problem, and naturally, means for solving this problem are neither disclosed nor suggested in any way.

In connection thereto, the direction in which the tear line extends is considered to depend on the ease of tearing of the seal parts. Thus, as a result of rigorous investigation by the inventors on the structure or configuration of the seal parts, it was discovered that the fused parts of the seal parts may include a thin part having a small relative thickness and a thick part having a large relative thickness, and the thin parts easily tear along the outlines thereof. Therefore, by specifying the arrangement of the thin parts, it is believed that continuous extension of the tear lines in the length direction can be promoted, whereby the seal parts can be more easily torn.

### [SOLUTION TO PROBLEM]

According to the present invention there is provided the pants-type absorbent article of independent claim 1. The dependent claims specify preferred but optional features.

According to the present disclosure, the following features are provided.

### [Aspect 1]

A pants-type absorbent article, comprising:
an abdominal-side part and a back-side part, and
seal parts which join end parts of the abdominal-side part in a width direction and end parts of the back-side part in the width direction, which are superimposed so that inner surfaces thereof face each other, to each other, wherein
the seal parts comprise a plurality of fused parts which fuse the end parts of the abdominal-side part in the width direction and the end parts of the back-side part in the width direction to each other, the fused parts being aligned in a length direction while being spaced apart from each other,
all of the fused parts each include a thin part having a small relative thickness and a thick part having a large relative thickness, all of the thin parts being arranged on an inner side of the fused parts in the width direction,
the fused parts each comprise a recessed groove, a peripheral wall and a bottom wall,
the bottom wall comprises the thin part having a small relative thickness and the thick part having a large relative thickness, and the small relative thickness and the large relative thickness are dimensions in a thickness direction of the absorbent article.

When a seal part is torn, in general, force is applied to the seal part from the inner side in the width direction. According to Aspect 1, since the thin parts of all of the fused parts are arranged on the inner side of the corresponding fused parts in the width direction, the seal part can be more easily torn, and thus, the pants-type absorbent article can be more easily removed from the wearer. Moreover, the seal parts are more easily torn along the entire length thereof.

### [Aspect 2]

The pants-type absorbent article according to Aspect 1, wherein
the seal parts join one of the end parts of the abdominal-side part in the width direction and one of the end parts of the back-side part in the width direction to each other, and another end part of the abdominal-side part in the width direction and another end part of the back-side part in the width direction to each other,
at least two mutually adjacent fused parts of the seal parts which join the one end parts of the abdominal-side part in the width direction and the back-side part in the width direction to each other each include a thin part having a small relative thickness and a thick part having a large relative thickness, all of the thin parts being arranged on the inner side of the fused parts in the width direction, and
at least two mutually adjacent fused parts of the seal parts which join the other end parts of the abdominal-side part in the width direction and the back side part in the width direction to each other each include a thin part having a small relative thickness and a thick part having a large relative thickness, all of the thin parts being arranged on the inner side of the fused parts in the width direction.

According to Aspect 2, the seal parts are more easily torn in any of the seal parts provided on the ends of the absorbent article in the width direction.

### [Aspect 3]

The pants-type absorbent article according to Aspects 1 or 2, wherein two mutually adjacent fused parts on upper end parts of the seal parts in the length direction each include a thin part having a small relative thickness and a thick part having a large relative thickness, all of the thin parts being arranged on the inner side of the fused part in the width direction.

According to Aspect 3, the starting of the tearing of the seal parts from the upper ends thereof in the length direction is made easier.

### [Aspect 4]

The pants-type absorbent article according to any one of Aspects 1 to 3, wherein two mutually adjacent fused parts on lower end parts of the seal parts in the length direction each include a thin part having a small relative thickness and a thick part having a large relative thickness, all of the thin parts being arranged on the inner side of the fused parts in the width direction.

According to Aspect 4, the starting of the tearing of the seal parts from the lower ends thereof in the length direction is made easier.

### [Aspect 5]

The pants-type absorbent article according to any one of Aspects 1 to 4, wherein
one or both of at least the end parts of the abdominal-side part in the width direction and at least the end parts of the back-side part in the width direction include a plurality of embossed parts provided with a predetermined pattern, and
a pitch of the fused parts in the length direction is less than a pitch of the embossed parts in the length direction.

When a tear line reaches the embossed part, the tear line may extend in substantially the width direction. According to Aspect 5, the frequency at which embossed parts are present in the gaps between the fused parts is limited, whereby the extension of the tear line in the width direction is limited.

### [Aspect 6]

The pants-type absorbent article according to any one of Aspects 1 to 5, wherein the pitch of the fused parts is less than 1 mm.

According to Aspect 6, the number of fused parts provided per unit length of the seal parts is increased, whereby the seal parts can be more easily torn.

### [Aspect 7]

The pants-type absorbent article according to any one of Aspects 1 to 6, wherein a dimension of an interval between the fused parts in the length direction is greater than a dimension of the fused parts in the length direction.

When a tear line extends through the gaps between the fused parts, the tear line may extend in substantially the width direction. According to Aspect 7, the dimension of the gaps in the length direction is reduced, and thus, tearing in substantially the width direction of the gaps is limited.

### [Aspect 8]

The pants-type absorbent article according to any one of Aspects 1 to 7, wherein the recessed grooves are arranged on side of the back-side part and the bottom walls are arranged on the side of the abdominal-side part.

### [Aspect 9]

The pants-type absorbent article according to any one of Aspects 1 to 7, wherein the recessed grooves are arranged on side of the abdominal-side part and the bottom walls are arranged on the side of the back-side part.

### [ADVANTAGEOUS EFFECTS OF INVENTION]

The seal parts can be easily torn, and thus the pants-type absorbent article can be easily removed from the wearer.

### BRIEF DESCRIPTION OF DRAWINGS

FIG. 1 is a perspective view of a disposable diaper in a worn state according to an embodiment of the present disclosure.
FIG. 2 is a plan view of the disposable diaper of FIG. 1 in a developed state.
FIG. 3 is a schematic view of the end parts of the abdominal-side part and the back-side part in the width direction as viewed from the width direction.
FIG. 4 is a partial plan view of the disposable diaper of FIG. 1 in an extended state.
FIG. 5(A) is a partial developed plan view of seal parts, and FIG. 5(B) is a schematic cross-sectional view of fused parts as viewed along line VB-VB of FIG. 5(A).
FIG. 6 is a schematic partial perspective view of the disposable diaper at the time of removal.
FIGS. 7(A) to 7(F) are schematic partial plan views showing tear lines in the seal parts.
FIG. 8 is a schematic partial plan view showing embossing of the back-side part.

### DESCRIPTION OF EMBODIMENTS

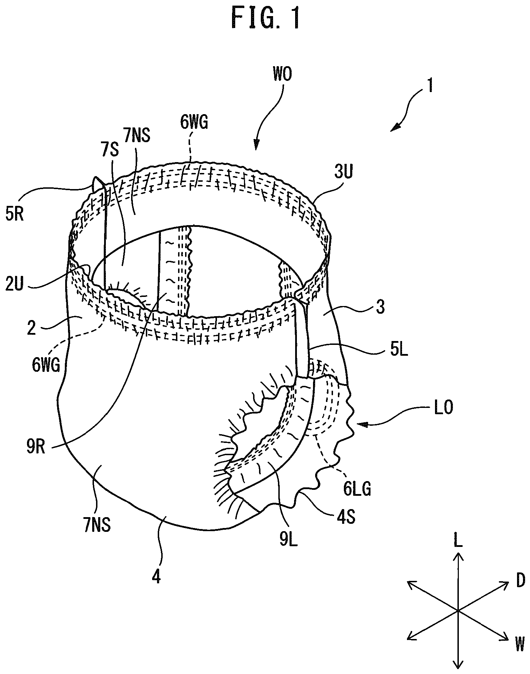

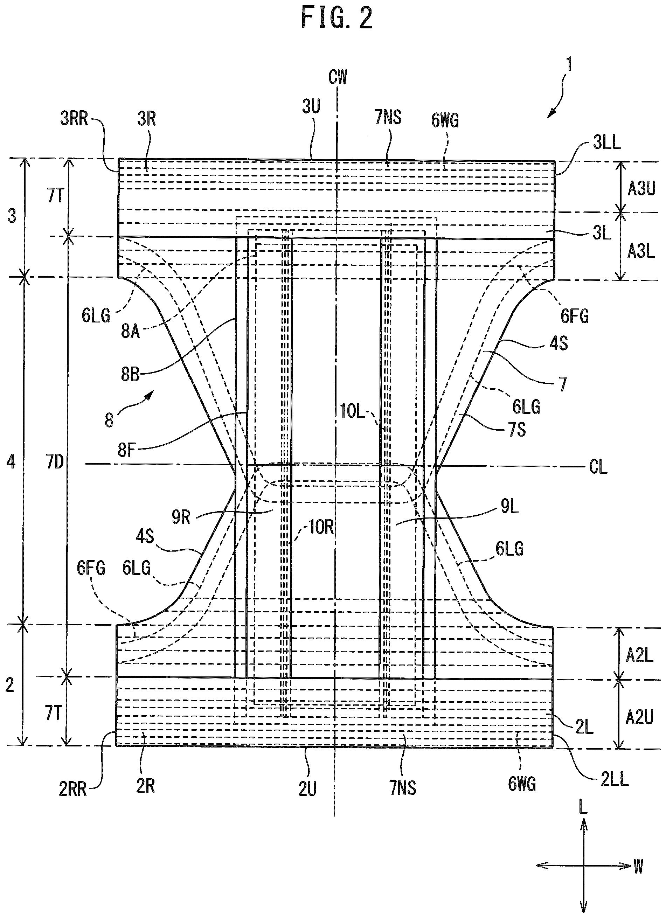

FIGS. 1 and 2 show a pants-type absorbent article 1 according to an embodiment of the present disclosure. The pants-type absorbent article 1 according to the embodiment of the present disclosure is constituted from a pants-type disposable diaper (hereinafter referred to simply as a "disposable diaper"). It should be noted that in FIG. 2, L represents a length direction of the disposable diaper 1 in a developed state, and W represents a width direction of the disposable diaper 1, which is perpendicular to the length direction L. Furthermore, in FIG. 2, CL represents a center line in the length direction which extends in the width direction W through a center of the disposable diaper 1 with respect to the length direction L, and CW represents a center line in the width direction which extends in the length direction L through a center of the disposable diaper 1 with respect to the width direction W. In this case, relative to the length direction L, a direction and side facing the center line CL in the length direction are an inner direction and inner side, and a direction and side away from the center line CL in the length direction are an outer direction and outer side, respectively. Relative to the width direction W, a direction and side facing the center line CW in the width direction are an inner direction and inner side, and a direction and side away from the center line CW in the width direction are an outer direction and the outer side. L in FIG. 1 represents the length direction of the disposable diaper 1 in a worn state. The downward direction and lower side in the length direction L in FIG. 1 correspond to the inner direction and inner side in the length direction L in FIG. 2, and the upward direction and upper side in the length direction L in FIG. 1 correspond to the outer direction and outer side in the length direction L in FIG. 2. Furthermore, D in FIG. 1 represents a depth direction of the disposable diaper 1.

Referring to FIG. 1, the disposable diaper 1 according to the embodiment of the present disclosure comprises an abdominal-side part 2, a back-side part 3, and an intermediate part 4 between the abdominal-side part 2 and the back-side part 3. Furthermore, the abdominal-side part 2 and the back-side part 3 are joined to each other by a pair of seal parts 5R, 5L which are arranged on the ends of the abdominal-side part 2 and the back-side part 3 in the width direction W. In this case, an upper edge 2U of the abdominal-side part 2 and an upper edge 3U of the back-side part 3 define a single waist opening WO through which a torso of a wearer passes. Furthermore, the side edges 4S, 4S of the intermediate part 4 in the width direction W define a pair of leg openings LO, LO through which legs of the wearer pass. It should be noted that when worn, the abdominal-side part 2 is arranged so as to face the abdomen of the wearer, the back-side 3 is arranged so as to face the back or buttocks of the wearer, and the intermediate part 4 is arranged so as to face the groin of the wearer.

In the embodiment of the present disclosure, in the developed state shown in FIG. 2, the abdominal-side part 2 and the back-side part 3 each form a roughly rectangular shape extending in the width direction W, and are spaced apart from each other in the length direction L. In the embodiment shown in FIG. 2, the upper edge 2U of the abdominal-side part 2 and the upper edge 3U of the back-side part 3 extend in the width direction W. Furthermore, in the embodiment shown in FIG. 2, outer edges 2LL, 2RR of the abdominal-side part 2 in the width direction W and outer edges 3LL, 3RR of the back-side part 3 in the width direction W extend in the length direction L. The intermediate part 4 is arranged between the abdominal-side part 2 and the back-side part 3. Side edges 4S, 4S of the intermediate part 4 in the width direction W are inwardly recessed in the width direction W. It should be noted that in the embodiment of the present disclosure, the abdominal-side part 2, the back-side part 3, and the intermediate part 4 are integrally formed. In another embodiment (not illustrated), the abdominal-side part 2, the back-side part 3, and the intermediate part 4 are separately formed.

In the embodiment of the present disclosure, a plurality of waist-gathering (WG) elastic members 6WG which extend in the width direction W while being spaced from each other in the length direction L are provided in an extended state in the upper area A2U, which is adjacent to the upper edge or outer edge 2U of the abdominal-side part 2, and in the upper area A3U, which is adjacent to the upper edge or outer edge 3U of the back-side part 3. The WG elastic members 6WG according to the embodiment of the present disclosure are provided between one outer edge 2LL in the width direction W and the other outer edge 2RR in the width direction W of the abdominal-side part 2, and between one outer edge 3LL in the width direction W and the other outer edge 3RR in the width direction W of the back-side part 3. Furthermore, a plurality of fit-gathering (FG) elastic members 6FG which extend in the width direction W while being spaced from each other in the length direction L are provided in an extended state in the lower area A2L, which is located inward in the length direction L with respect to the upper area A2U of the abdominal-side part 2, and the lower area A3L, which is located inward in the length direction L with respect to the upper area A3U of the back-side part 3. The FG elastic members 6FG according to the embodiment of the present disclosure are provided between one outer edge 2LL in the width direction W and the other outer edge 2RR in the width direction W of the abdominal-side part 2 and between one outer edge 3LL in the width direction W and the other outer edge 3RR in the width direction W of the back-side part 3. Further, a plurality of leg-gathering (LG) elastic members 6LG are provided in an extended state while being spaced from each other in a direction perpendicular to the direction of extension. The LG elastic members 6LG include those which extend from one outer edge 2LL of the abdominal-side part 2 in the width direction W, which is included in the lower area A2L of the abdominal-side part 2, along one side edge 4S of the intermediate part 4, next extend in the width direction W at the center in the width direction W, then extend along the other side edge 4S of the intermediate part 4, and reach the other outer edge 2RR of the abdominal-side part 2 in the width direction W, which is included in the lower area A2L of the abdominal-side part 2, and those which extend from one outer edge 3LL of the back-side part 3 in the width direction W, which is included in the lower area A3L of the back-side part 3, along one side edge 4S of the intermediate part 4, next extends in the width direction W at the center in the width direction W, then extends along the other side edge 4S of the intermediate part 4, and reaches the other outer edge 3RR of the back-side part 3 in the width direction W, which is included in the lower area A3L of the back-side part 3.

Tensile stresses in the width direction W are exerted mainly by the WG elastic members 6WG in the upper areas A2U, A3U. Tensile stresses in the width direction W are exerted mainly by the FG elastic members 6FG in the lower areas A2L, A3L. In the embodiment of the present disclosure, the tensile stresses in the width direction W in the upper areas A2U, A3U are greater than the tensile stresses in the width direction W in the lower areas A2L, A3L. In order to achieve these stresses, the extension ratios, the numbers, thicknesses, cross-sectional shapes, etc., of the WG elastic members 6WG, the FG elastic members 6FG, and the LG elastic members 6LG are adjusted.

In an example, a plurality of 620 dtx elastic threads having extension ratios of 2.8 times are provided at 6 mm intervals as the WG elastic members 6WG. A plurality of 470 to 940 dtx elastic threads having extension ratios of 2.1 to 2.7 times are provided at 7 to 15 mm intervals as the FG elastic members 6FG. A plurality of 620 dtx elastic fibers having extension ratios of 2.0 times are provided at 7.5 mm intervals as the LG elastic members 6LG.

In the embodiment of the present disclosure, the abdominal-side part 2, the back-side part 3, and the intermediate part 4 are formed from a liquid-impermeable cover sheet 7. The cover sheet 7 according to the embodiment of the present disclosure comprises a skin-side sheet 7S and a non-skin-side sheet 7NS which are laminated on and adhered to each other. In a worn state, the skin-side sheet 7S is arranged on the side near the wearer or inner side, and the non-skin-side sheet 7NS is arranged on the side far from the wearer or the outer side. Furthermore, in the embodiment of the present disclosure, the non-skin-side sheet 7NS is folded back onto the skin-side sheet 7S in both outer ends in the length direction L. As a result, the outer side portions of the non-skin-side sheet 7 in the length direction L form three-layer portions 7T, 7T overlapped in the order of non-skin-side sheet 7NS, skin-side sheet 7S, and non-skin-side sheet 7NS. The three-layer portions 7T, 7T are included in the abdominal-side part 2 and the back-side part 3. A two-layer portion 7D in which the skin-side sheet 7S and the non-skin-side sheet 7NS are overlapped is formed at the central portion of the cover sheet 7 in the length direction L. The elastic members 6WG, 6FG, and 6LG are connected between the skin-side sheet 7S and the non-skin-side sheet 7NS.

The cover sheet 7 is formed from an arbitrary liquid-impermeable sheet such as an impermeable nonwoven fabric or synthetic resin film, a composite sheet thereof, an SB nonwoven fabric, or an SMS nonwoven fabric formed from a polyolefin-based material such as, for example, polypropylene or polyethylene. Furthermore, the basis weight of the cover sheet 7 is, for example 5 to 100 g/m², preferably 10 to 50 g/m². The thickness of the cover sheet 7 is, for example, 0.2 to 5 mm, preferably 0.2 to 2 mm. In another embodiment (not illustrated), the cover sheet 7 is constituted from a single sheet. In yet another embodiment (not illustrated), the entirety of the cover sheet 7 is formed from a two-layer portion without being provided with three-layer portions.

The disposable diaper 1 according to the embodiment of the present disclosure further comprises an absorbent body 8 provided on the inner surface of the cover sheet 7. The absorbent body 8 comprises a liquid-permeable front sheet 8F, a liquid-impermeable back sheet 8B, and an absorbent 8A arranged between the front sheet 8F and the back sheet 8B. The absorbent 8A according to the embodiment of the present disclosure comprises, in the present embodiment, an absorbent core and a core wrapper which envelopes the absorbent core. The absorbent 8A is connected with both the front sheet 8F and the back sheet 8B, and the front sheet 8F and the back sheet 8B are connected in the edge portions thereof. The front sheet 8F is formed from, for example, a liquid-permeable non-woven fabric or woven fabric, a synthetic resin film having liquid-penetration holes, or a composite sheet thereof. The back sheet 8B is formed from, for example, a liquid-impermeable non-woven fabric, a synthetic resin film, a composite sheet thereof, or an SMS non-woven fabric. The absorbent 8A is formed from pulp fibers, synthetic fibers, or a water-absorbing polymer.

The disposable diaper 1 according to the embodiment of the present disclosure further comprises liquid-impermeable leak barriers 9R, 9L. The leak barriers 9R, 9L are arranged along the length direction L on the sides of the front sheet 8F in the width direction W. Elastic members 10R, 10L which extend in the length direction L are arranged on the tips of the leak barriers 9R, 9L, respectively.

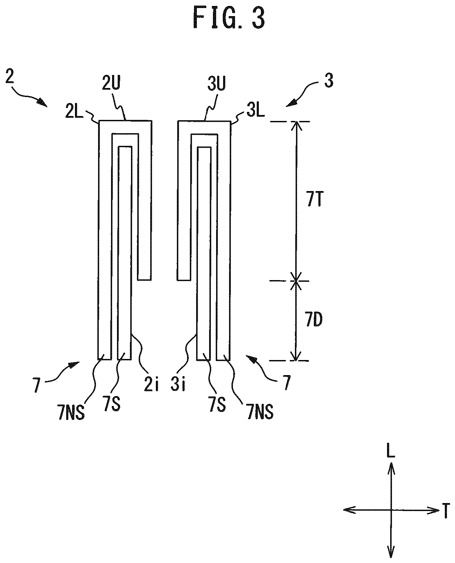

In the embodiment of the present disclosure, the disposable diaper 1 in a developed state is folded along, for example, the center line CL in the length direction L so that the inner surface 2i of the abdominal-side part 2 and the inner surface 3i of the back-side part 3 face each other. In this case, one end 2L of the abdominal-side part 2 in the width direction W and one end 3L of the back-side part 3 in the width direction W are superimposed, and the other end 2R of the abdominal-side part 2 in the width direction W and the other end 3R of the back-side part 3 in the width direction W are superimposed. FIG. 3 schematically illustrates one end 2L of the abdominal-side part 2 in the width direction W and one end 3L of the back-side part 3 in the width direction W, which are superimposed. It should be noted that in FIG. 3, T represents a thickness direction of the disposable diaper 1. Next, a seal part 5L which joins one end 2L of the abdominal-side part 2 in the width direction W and one end 3L of the back-side part 3 in the width direction W to each other is provided, and a seal part 5R which joins the other end 2R of the abdominal-side part 2 in the width direction W and the other end 3R of the back-side part 3 in the width direction W to each other is provided. Furthermore, in the embodiment of the present disclosure, adhesive is applied between the ends 2L, 2R of the abdominal-side part 2 in the width direction W and the ends 3L, 3R of the back-side part 3 in the width direction W. A hot-melt adhesive is an example of the adhesive.

Next, the seal parts 5L, 5R according to the embodiment of the present disclosure will be further described. The disposable diaper 1 according to the embodiment of the present disclosure is formed so as to be substantially symmetrical with respect to the center line CW in the width direction W. Thus, the seal part 5L and the seal part 5R have substantially identical structures. The seal part 5L will be described below.

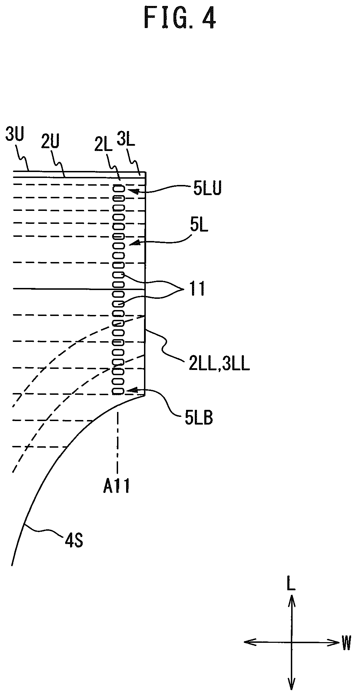

FIG. 4 is a partial plan view of the disposable diaper 1 according to the present disclosure in an extended state. As shown in FIG. 4, the seal part 5L comprises a plurality of fused parts 11. The fused parts 11 according to the embodiment of the present disclosure are aligned along the axis A11 extending in the length direction L (including the case of slight inclination in the length direction L) while being spaced from each other in the length direction L. Furthermore, the fused parts 11 according to the embodiment of the present disclosure are provided along substantially the entire length in the length direction L of the end 2L of the abdominal-side part 2 in the width direction W and the end 2R of the back-side part 3 in the width direction W. It should be noted that the outer edge 2LL of the abdominal-side part 2 in the width direction W and the outer edge 3LL of the back-side part 3 in the width direction W are substantially aligned with each other with respect to the width direction W.

The fused parts 11 according to the embodiment of the present disclosure are formed by a fused part forming device (not illustrated). The fused part forming device comprises, for example, an anvil roller having protrusions on the outer circumferential surface thereof, and an energy application member which is arranged so as to face the outer circumferential surface of the anvil roller. When ultrasonic energy is used to form the fused parts, the energy application member is formed from an ultrasonic horn. The method for the production of the disposable diaper 1 according to the embodiment of the present disclosure will be described briefly. First, a continuous body in which a plurality of disposable diapers 1 in a developed state are connected in the width direction W is prepared. Next, the continuous body is folded along the center line CL in the length direction L, and abdominal-side parts 2 and the back-side parts 3 are superimposed on each other so that the inner surfaces 2i, 3i thereof face each other. Next, the continuous body is conveyed along the outer circumferential surface of the anvil roller. Thereafter, the ultrasonic horn is moved toward the anvil roller, ultrasonic energy is imparted to the continuous body by the ultrasonic horn while the continuous body is compressed by the ultrasonic horn and the protrusions of the anvil roller, whereby the fused parts 11 are formed. Next, the continuous body is cut into several disposable diapers 1. In another embodiment (not illustrated), the fused part forming device forms the fused parts by imparting heat energy to the continuous body.

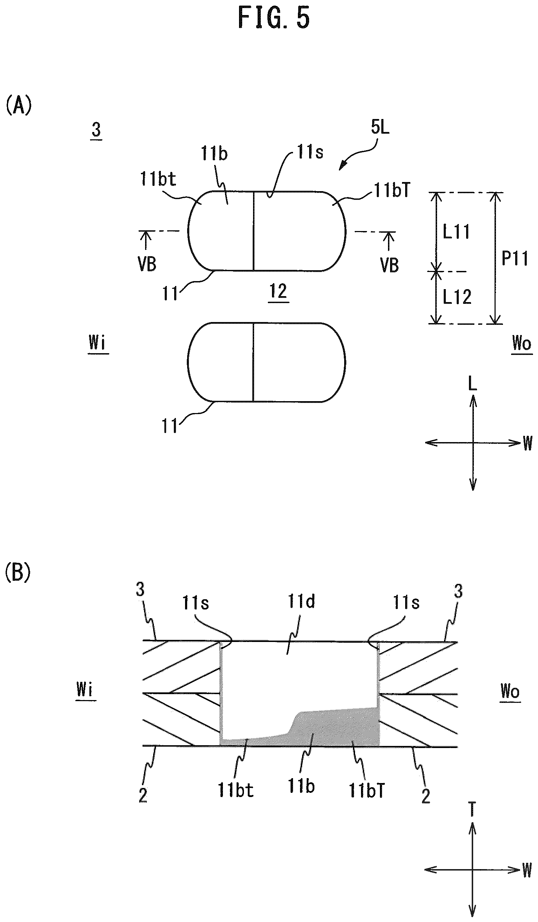

FIG. 5(A) schematically illustrates a plan view of the fused parts 11 according to the embodiment of the present disclosure, and FIG. 5(B) schematically illustrates a cross-sectional view of the fused parts 11 according to the embodiment of the present disclosure as viewed along line VB-VB of FIG. 5(A). In FIGS. 5(A) and 5(B), Wi represents an inner side in the width direction W, and Wo represents an outer side in the width direction W. The fused parts 11 according to the embodiment of the present disclosure have oval outlines which extend in the width direction W, and comprise recessed grooves 11d, cylindrical peripheral wall parts 11s, and plate-like bottom wall parts 11b. In the example shown in FIGS. 5(A) and 5(B), the recessed grooves 11d are arranged on the back-side part 3 side, and the bottom wall parts 11b are arranged on the abdominal-side part 2 side. In another embodiment (not illustrated), the recessed grooves 11d are arranged on the side of the abdominal-side part 2 and the bottom wall parts 11b are arranged on the side of the back-side part 3.

Furthermore, in the embodiment of the present disclosure, the pitch P11 of the fused parts 11 in the length direction L is less than 1 mm. Further, in the embodiment of the present disclosure, the gaps between the fused parts 11 or the lengths L12 of the non-fused parts 12 is less than the lengths (dimension in the length direction L) L11 of the fused parts 11.

The bottom wall part 11b is formed by melting and subsequently solidifying at least one of the abdominal-side part 2 and the back-side part 3, which are interposed between the tip of the ultrasonic horn of the fused part forming device described above and the tips of the protrusions of the anvil roller. The peripheral wall part 11s is formed by melting and subsequently solidifying at least one of the abdominal-side part 2 and the back-side part 3, which are in contact with the sides of the protrusion of the anvil roller.

In the embodiment of the present disclosure, the bottom wall 11b comprises a thin part 11bt which has a small relative thickness (dimension in the thickness direction T), and a thick part 11bT which has a large relative thickness. In the embodiment of the present disclosure, the thickness of the thin part 11bt is 100 µm or less, the thickness of the thick part 11bT is 60 µm or more, and the difference between the thickness of the thick part 11bT and the thickness of the thin part 11bt is 20 µm or more. It should be noted that the average thickness of the thin part 11bt in the width direction W is used as the thickness of the thin part 11bt. Furthermore, the average thickness of the thick part 11bT in the width direction W is used as the thickness of the thick part 11bT. In the embodiment of the present disclosure, the thin part 11bt is arranged on the inner side Wi of the corresponding fused part 11 in the width direction W, and the thick part 11bT is arranged on the outer side Wo of the corresponding fused part 11 in the width direction W. In other words, in the embodiment of the present disclosure, the thickness of the bottom wall part 11b is not constant in the width direction W. Conversely, the thickness of the bottom wall part 11b is constant (including substantially constant) in the length direction L.

The thin part 11bt has a degree of fusing or compression which is high as compared to the thick part 11bT. Thus, in an example, the thin part 11bt is formed by making the amount of energy (the product of the amount of energy applied per unit time and the time during which energy is applied) imparted by the fused part forming device to the abdominal-side part 2 and the back-side part 3 relatively large, and the thick part 11bT is formed by making the amount of energy imparted by the fused part forming device to the abdominal-side part 2 and the back-side part 3 relatively small. In another example, the thin part 11bt is formed by making the gap between the ultrasonic horn and the protrusions of the anvil roller relatively small, and the thick part 11bT is formed by making the gap between the ultrasonic horn and the protrusions of the anvil roller relatively large. The size of the gap between the ultrasonic horn and the protrusions of the anvil roller can be adjusted by, for example, changing the shapes of the protrusions of the anvil roller.

In the embodiment of the present disclosure, all of the fused parts 11 included in the seal part 5L comprise thin parts 11bt arranged on the inner side in the width direction W and thick parts 11bT arranged on the outer side in the width direction W.

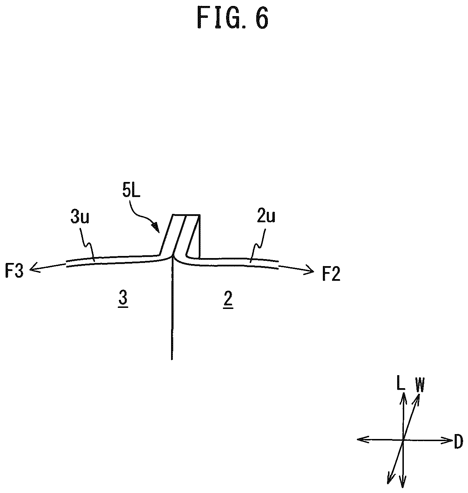

In the embodiment of the present disclosure, in order to remove the disposable diaper 1 from the body of the wearer, the wearer or a caregiver manually rips the seal parts 5, whereby the abdominal-side part 2 and the back-side part 3 are separated from each other. For example, as shown in FIG. 6, the wearer or a caregiver holds the abdominal-side part 2 around the upper edge 2U and the back-side part 3 around the upper edge 3U, and pulls in the directions of arrows F2 and F3, respectively, whereby the seal part 5L is torn from substantially the upper part in the length direction L toward the lower part in the length direction L. In this case, arrow F2 is directed substantially forward in the depth direction D, and arrow F3 is directed substantially backwards in the depth direction D. As a result, force is applied to the inner side portions of the fused parts 11 of the seal part 5L in the width direction W.

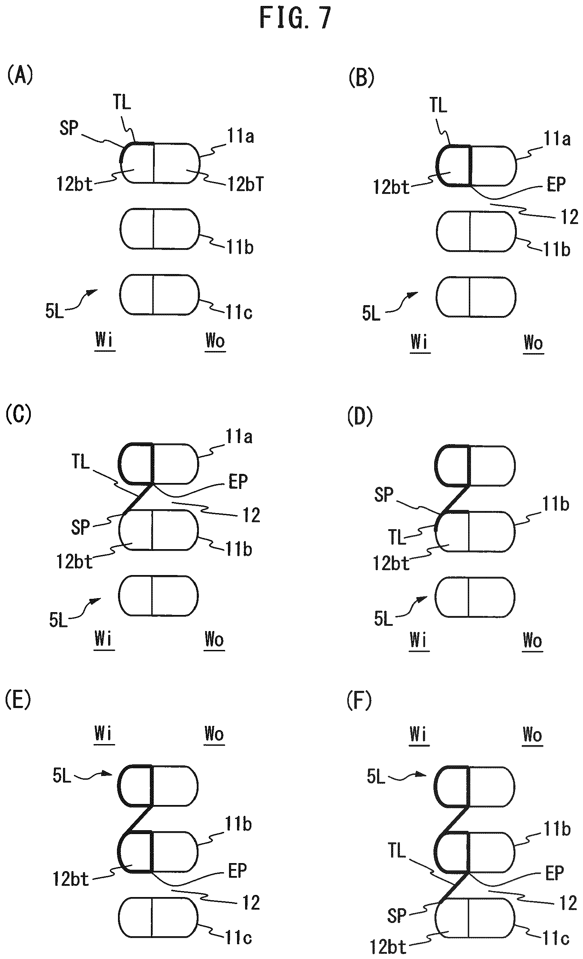

The thin part 11bt of the fused part 11 is a portion in which high-degree fusing has been performed, as described above, and the thin part 11bt itself is relatively hard. Thus, the seal part 5L tears more easily along the outline of the thin part 11bt, rather than the thin part 11bt itself tearing. Thus, when the wearer or a caregiver tears the seal part 5L, a tear line TL is formed along the outline of the fused part 11a arranged uppermost in the length direction L among the fused parts 11 of the seal part 5L, as shown in, for example, FIG. 7(A). More specifically, the tear line TL is formed at the starting point SP of the outline of the fused part 11a that is located on the inner side in the width direction W and on the upper side in the length direction L.

Next, the tear line TL advances along the outline of the thin part 11bt of the fused part 11a, thereafter further progresses in substantially the length direction L so as to surround the entire periphery of the thin part 11bt, and reaches the end point EP located on the lower side of the fused part 11a in the length direction L, as shown in FIG. 7(B).

Next, the tear line TL progresses from the end point EP of the fused part 11a in substantially the length direction L through the gap 12 between the fused part 11a and the subsequent fused part 11b, as shown in FIG. 7(C), and reaches the starting point SP of the fused part 11b located on the inner side in the width direction W and on the upper side length direction L. In other words, the gap 12 tears along the tear line TL. In this case, the back-side part 3 may tear without tearing of the abdominal-side part 2 in some cases, the abdominal-side part 2 may tear without tearing of the back-side part 3 in some cases, or the abdominal-side part 2 and the back-side part 3 may tear in some cases.

Next, the tear line TL progresses along the outline of the thin part 11bt of the fused part 11b, as shown in FIG. 7(D). Thereafter, the tear line TL further progresses in substantially the length direction L so as to surround the entire periphery of the thin part 11bt of the fused part 11b, as shown in FIG. 7(E), and reaches the end point EP located on the lower side of the fused part 11b in the length direction L.

Next, the tear line TL progresses from the end point EP of the fused part 11b in substantially the length direction L through the gap 12 between the fused part 11b and the subsequent fused part 11c, as shown in FIG. 7(F), and reaches the starting point SP of the fused part 11c located on the inner side in the width direction W and on the upper side in the length direction L. Next, the tear line TL progresses along the outline of the thin part 11bt of the fused part 11c.

The tear line TL progresses sequentially alternating between the outlines or outer peripheries of the thin parts 11bt and gaps 12 in this manner. As a result, progress of the tear line TL is promoted in substantially the length direction L, and the progress in substantially the width direction W is restricted. Thus, the seal part 5L is easily torn and the disposable diaper 1 is easily removed from the wearer.

The ease of progression of the tear line TL in substantially the length direction L in this manner is due to the alignment of the thin parts 11bt of the fused parts 11 on the inner side in the width direction W. In the embodiment of the present disclosure, all of the fused parts 11 included in the seal part 5L comprise thin parts 11bt arranged on the inner side in the width direction W and thick parts 11bT arranged on the outer side in the width direction W, as described above. As a result, the seal part 5L is more easily torn along the entire in the length direction L thereof. Thus, the disposable diaper 1 is easily removed from the wearer.

In connection thereto, according to another point of view, in the embodiment of the present disclosure, two mutually adjacent fused parts 11 in the upper end part 5LU (FIG. 4) of the seal part 5L in the length direction L include thin parts 11bt arranged on the inner side in the width direction W and thick parts 11bT arranged on the outer side in the width direction W. As a result, the starting of the tearing of the seal part 5L from the upper end part 5LU in the length direction L is made easier.

According to yet another point of view, in the embodiment of the present disclosure, two mutually adjacent fused parts 11 in the lower end part 5LB (FIG. 4) of the seal part 5L in the length direction L include a thin part 11bt arranged on the inner side in the width direction W and a thick part 11bT arranged on the outer side in the width direction W. As a result, the starting of the tearing of the seal part 5L from the lower end part 5LB in the length direction L is made easier.

According to yet another point of view, in the embodiment of the present disclosure, two mutually adjacent fused parts 11 in the upper part 5LU (FIG. 4) of the seal part 5L in the length direction L include thins part 11bt arranged on the inner side in the width direction W and thick parts 11bT arranged on the outer side in the width direction W, and two mutually adjacent fused parts 11 on the lower end part 5LB (FIG. 4) of the seal part 5L in the length direction L include thin parts 11bt arranged on the inner side in the width direction W and thick parts 11bT arranged on the outer side in the width direction W. As a result, the tearing of the seal part 5L is made easy regardless of whether the tearing of the seal part 5L starts from the upper end part 5LU in the length direction L or from the lower end part 5LB in the length direction L.

In connection thereto, the seal part 5L is more easily torn over the entire thereof in the length direction L.

In the embodiment of the present disclosure, the pitch P11 of the fused parts 11 is less than 1 mm, as described above. As a result, the number of fused parts provided per unit length of the seal part 5L is increased. Thus, the seal part 5L is torn more easily.

The tear line TL is more likely to extend in substantially the width direction W when the tear line TL progresses through the gap or non-fused part 12 than when the tear line TL progresses through the surrounding of the thin part 11bt of the fused part 11. In the embodiment of the present disclosure, the length L12 of the gap 12 is less than the length L11 of the fused part 11, as described above. As a result, progression of the tear line TL in substantially the width direction W in the gaps 12 is more limited.

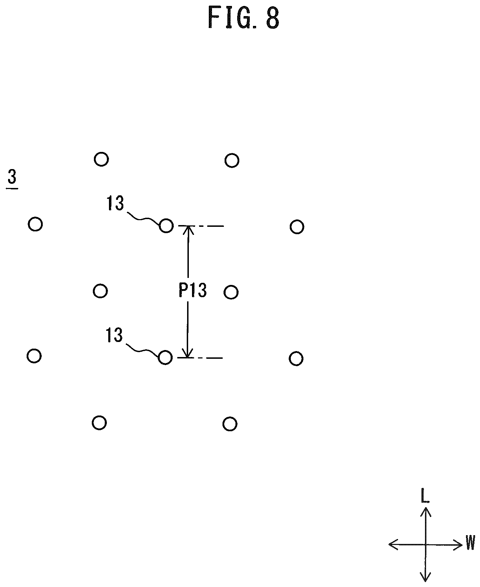

In the embodiment of the present disclosure, a plurality of embossed parts provided in a predetermined pattern are provided on one or both of at least the end 2L of the abdominal-side part 2 in the width direction W and at least the end 3L of the back-side part 3 in the width direction W. The embossed parts 13 according to the embodiment of the present disclosure are provided on the entirety of the abdominal-side part 2 and the entirety of the back-side part 3. FIG. 8 shows an example of the pattern of the embossed parts 13 provided on the back-side part 3. When the tear line TL reaches the embossed part 13, the direction of progression of the tear line TL changes, and the tear line TL may progress in substantially the width direction W. In the embodiment of the present disclosure, the pitch P11 (FIG. 5) of the fused parts 11 in the length direction L is less than the pitch P13 of the embossed parts 13 in the length direction L. As a result, the frequency or probability of the presence of the embossed parts 13 in the gaps 12 is limited. Thus, the progression of the tear line TL in substantially the width direction W is limited.

In connection thereto, the seal part 5L according to the embodiment of the present disclosure is relatively difficult to tear along the outline of the thick part 11bT. Thus, when the disposable diaper 1 is worn, the seal part 5L continues to reliably join the abdominal-side part 2 and the back-side part 3. In other words, the seal part 5L according to the embodiment of the present disclosure is comparatively difficult to tear when the disposable diaper 1 is worn but is comparatively easy to tear during removal of the disposable diaper 1.

The description of the seal part 5L above also applies to the seal part 5R. According to another point of view, in the embodiment of the present disclosure, in the seal part 5L, two mutually adjacent fused parts 11 include thin parts 11bt arranged on the inner side in the width direction W and thick parts 11bT arranged on the outer side in the width direction W, and in the seal part 5R, two mutually adjacent fused parts 11 include thin parts 11bt arranged on the inner side in the width direction W and thick parts 11bT arranged on the outer side in the width direction W. As a result, whether the seal part 5L is torn or the seal part 5R is torn, the disposable diaper 1 is easily removed from the wearer.

Furthermore, in the various embodiments of the present disclosure described above, the ends of the abdominal-side part 2 and the back-side part 3 in the width direction W are joined to each other by a pair of seal parts 5L, 5R. In another embodiment (not illustrated), one end of the abdominal-side part 2 in the width direction W and one end of the back-side part 3 in the width direction W are joined to each other by a seal part, and the other ends of the abdominal-side part 2 and the back-side part 3 in the width direction W are joined to each other by means other than a seal part.

### REFERENCE SIGNS LIST

- 1: disposable diaper
- 2: abdominal-side part
- 3: back-side part
- 5L, 5R: seal part
- 11: fused part
- 11bt: thin part
- 11bT: thick part

## Claims

1. A pants-type absorbent article (1), comprising:
an abdominal-side part (2) and a back-side part (3), and
seal parts (5L, 5R) which join end parts (2L, 2R) of the abdominal-side part (2) in a width direction (W) and end parts (3L, 3R) of the back-side part (3) in the width direction (W), which are superimposed so that inner surfaces (2i,3i) thereof face each other, to each other,
wherein
the seal parts (5L, 5R) comprise a plurality of fused parts (11) which fuse the end parts (2L, 2R) of the abdominal-side part (2) in the width direction (W) and the end parts (3L, 3R) of the back-side part (3) in the width direction (W) to each other, the fused parts (11) being aligned in a length direction (L) while being spaced apart from each other,
all of the fused parts (11) each include a thin part (11bt) having a small relative thickness and a thick part (11bT) having a large relative thickness, all of the thin parts (11bt) being arranged on an inner side of the fused parts (11) in the width direction (W),
the fused parts (11) each comprise a recessed groove (11d), a peripheral wall (11s) and a bottom wall (11b),
the bottom wall (11b) comprises the thin part (11bt) having a small relative thickness and the thick part (11bT) having a large relative thickness, and
the small relative thickness and the large relative thickness are dimensions in a thickness direction (T) of the absorbent article (1).

2. The pants-type absorbent article (1) according to claim 1, wherein
the seal parts (5L, 5R) join one of the end parts (2L) of the abdominal-side part (2) in the width direction (W) and one of the end parts (3L) of the back-side part (3) in the width direction (W) to each other, and another end part (2R) of the abdominal-side part (2) in the width direction (W) and another end part (3R) of the back-side part (3) in the width direction (W) to each other,
at least two mutually adjacent fused parts (11) of the seal parts (5L) which join the one end parts (2L, 3L) of the abdominal-side part (2) and the back-side part (3) in the width direction (W) to each other each include the thin part (11bt) having a small relative thickness and the thick part (11bT) having a large relative thickness, all of the thin parts (11bt) being arranged on the inner side (Wi) of the fused parts (11) in the width direction (W), and
at least two mutually adjacent fused parts (11) of the seal parts (5R) which join the other end parts (2R, 3R) of the abdominal-side part (2) and the back side part in the width direction (W) to each other each include the thin part (11bt) having a small relative thickness and the thick part (11bT) having a large relative thickness, all of the thin parts (11bt) being arranged on the inner side of the fused parts (11) in the width direction (W).

3. The pants-type absorbent article (1) according to claim 1 or 2, wherein two mutually adjacent fused parts (11) on upper end parts (5LU, 5RU) of the seal parts (5L, 5R) in the length direction (L) each include the thin part (11bt) having a small relative thickness and the thick part (11bT) having a large relative thickness, all of the thin parts (11bt) being arranged on the inner side (Wi) of the fused part in the width direction (W).

4. The pants-type absorbent article (1) according to any one of claims 1 to 3, wherein two mutually adjacent fused parts (11) on lower end parts (5LB, 5RB) of the seal parts (5L, 5R) in the length direction (L) each include the thin part (11bt) having a small relative thickness and the thick part (11bT) having a large relative thickness, all of the thin parts (11bt) being arranged on the inner side of the fused parts (11) in the width direction (W).

5. The pants-type absorbent article (1) according to any one of claims 1 to 4, wherein
one or both of at least the end parts (2L, 2R) of the abdominal-side part (2) in the width direction (W) and at least the end parts (3L, 3R) of the back-side part (3) in the width direction (W) include a plurality of embossed parts (13) provided with a predetermined pattern, and
a pitch (P11) of the fused parts (11) in the length direction (L) is less than a pitch (P13) of the embossed parts (13) in the length direction (L).

6. The pants-type absorbent article (1) according to any one of claims 1 to 5, wherein the pitch (P11) of the fused parts (11) is less than 1 mm.

7. The pants-type absorbent article (1) according to any one of claims 1 to 6, wherein a dimension (L12) of an interval (12) between the fused parts (11) in the length direction (L) is greater than a dimension (L11) of the fused parts (11) in the length direction (L).

8. The pants-type absorbent article (1) according to any one of claims 1 to 7, wherein the recessed grooves (11d) are arranged on side of the back-side part (3) and the bottom walls (11b) are arranged on the side of the abdominal-side part (2).

9. The pants-type absorbent article (1) according to any one of claims 1 to 7, wherein the recessed grooves (11d) are arranged on side of the abdominal-side part (2) and the bottom walls (11b) are arranged on the side of the back-side part (3).

## Patentansprüche

1. Höschenartiger absorbierender Artikel (1), der Folgendes umfasst:
ein Bauchseitenteil (2) und ein Rückenseitenteil (3) und
Verschlussteile (5L, 5R), die Endteile (2L, 2R) des Bauchseitenteils (2) in der Breitenrichtung (W) und Endteile (3L, 3R) des Rückenseitenteils (3) in der Breitenrichtung (W) verbinden, die überlagert sind, sodass innere Oberflächen (2i, 3i) davon einander, zueinander, zugewandt sind, wobei
die Verschlussteile (5L, 5R) eine Vielzahl von verschmolzenen Teilen (11) umfassen, die die Endteile (2L, 2R) des Bauchseitenteils (2) in der Breitenrichtung (W) und die Endteile (3L, 3R) des Rückenseitenteils (3) in der Breitenrichtung (W) miteinander verschmelzen, wobei die verschmolzenen Teile (11) in einer Längsrichtung (L) ausgerichtet sind, während sie voneinander beabstandet sind,
alle der verschmolzenen Teile (11) jeweils ein dünnes Teil (11bt), das eine kleine relative Dicke aufweist, und ein dickes Teil (11bT), das eine große relative Dicke aufweist, einschließen, wobei alle der dünnen Teile (11bt) auf einer Innenseite der verschmolzenen Teile (11) in der Breitenrichtung (W) angeordnet sind,
die verschmolzenen Teile (11) jeweils eine ausgesparte Vertiefung (11d), eine umlaufende Wand (11s) und eine Bodenwand (11b) umfassen,
die Bodenwand (11b) das dünne Teil (11bt), das eine kleine relative Dicke aufweist, und das dicke Teil (11bT), das eine große relative Dicke aufweist, umfasst und
die kleine relative Dicke und die große relative Dicke Abmessungen in einer Dickenrichtung (T) des absorbierenden Artikels (1) sind.

2. Höschenartiger absorbierender Artikel (1) nach Anspruch 1, wobei
die Verschlussteile (5L, 5R) eines der Endteile (2L) des Bauchseitenteils (2) in der Breitenrichtung (W) und eines der Endteile (3L) des Rückenseitenteils (3) in der Breitenrichtung (W) miteinander verbinden und ein anderes Endteil (2R) des Bauchseitenteils (2) in der Breitenrichtung (W) und ein anderes Endteil (3R) des Rückenseitenteils (3) in der Breitenrichtung (W) miteinander verbinden,
mindestens zwei aneinander angrenzende verschmolzene Teile (11) der Verschlussteile (5L), die die einen Endteile (2L, 3L) des Bauchseitenteils (2) und des Rückenseitenteils (3) in der Breitenrichtung (W) miteinander verbinden, jeweils das dünne Teil (11bt), das eine kleine relative Dicke aufweist, und das dicke Teil (11bT), das eine große relative Dicke aufweist, einschließen, wobei alle der dünnen Teile (11bt) auf der Innenseite (Wi) der verschmolzenen Teile (11) in der Breitenrichtung (W) angeordnet sind, und
mindestens zwei aneinander angrenzende verschmolzene Teile (11) der Verschlussteile (5R), die die anderen Endteile (2R, 3R) des Bauchseitenteils (2) und des Rückenseitenteils in der Breitenrichtung (W) miteinander verbinden, jeweils das dünne Teil (11bt), das eine kleine relative Dicke aufweist, und das dicke Teil (11bT), das eine große relative Dicke aufweist, einschließen, wobei alle der dünnen Teile (11bt) auf der Innenseite der verschmolzenen Teile (11) in der Breitenrichtung (W) angeordnet sind.

3. Höschenartiger absorbierender Artikel (1) nach Anspruch 1 oder 2, wobei zwei aneinander angrenzende verschmolzene Teile (11) auf oberen Endteilen (5LU, 5RU) der Verschlussteile (5L, 5R) in der Längsrichtung (L) jeweils das dünne Teil (11bt), das eine kleine relative Dicke aufweist, und das dicke Teil (11bT), das eine große relative Dicke aufweist, einschließen, wobei alle der dünnen Teile (11bt) auf der Innenseite (Wi) des verschmolzenen Teils (11) in der Breitenrichtung (W) angeordnet sind.

4. Höschenartiger absorbierender Artikel (1) nach einem der Ansprüche 1 bis 3, wobei zwei aneinander angrenzende verschmolzene Teile (11) auf unteren Endteilen (5LB, 5RB) der Verschlussteile (5L, 5R) in der Längsrichtung (L) jeweils das dünne Teil (11bt), das eine kleine relative Dicke aufweist, und das dicke Teil (11bT), das eine große relative Dicke aufweist, einschließen, wobei alle der dünnen Teile (11bt) auf der Innenseite der verschmolzenen Teile (11) in der Breitenrichtung (W) angeordnet sind.

5. Höschenartiger absorbierender Artikel (1) nach einem der Ansprüche 1 bis 4, wobei
eines oder beide von mindestens den Endteilen (2L, 2R) des Bauchseitenteils (2) in der Breitenrichtung (W) und mindestens den Endteilen (3L, 3R) des Rückenseitenteils (3) in der Breitenrichtung (W) eine Vielzahl von geprägten Teilen (13) einschließen, die mit einem vorgegebenen Muster bereitgestellt sind, und
eine Ganghöhe (P11) der verschmolzenen Teile (11) in der Längsrichtung (L) kleiner ist als eine Ganghöhe (P13) der geprägten Teile (13) in der Längsrichtung (L).

6. Höschenartiger absorbierender Artikel (1) nach einem der Ansprüche 1 bis 5, wobei die Ganghöhe (P11) der verschmolzenen Teile (11) weniger als 1 mm beträgt.

7. Höschenartiger absorbierender Artikel (1) nach einem der Ansprüche 1 bis 6, wobei eine Abmessung (L12) eines Abstands (12) zwischen den verschmolzenen Teilen (11) in der Längsrichtung (L) größer ist als eine Abmessung (L11) der verschmolzenen Teile (11) in der Längsrichtung (L).

8. Höschenartiger absorbierender Artikel (1) nach einem der Ansprüche 1 bis 7, wobei die ausgesparten Vertiefungen (11d) auf der Seite des Rückseitenteils (3) angeordnet sind und die Bodenwände (11b) auf der Seite des Bauchseitenteils (2) angeordnet sind.

9. Höschenartiger absorbierender Artikel (1) nach einem der Ansprüche 1 bis 7, wobei die ausgesparten Vertiefungen (11d) auf der Seite des Bauchseitenteils (2) angeordnet sind und die Bodenwände (11b) auf der Seite des Rückseitenteils (3) angeordnet sind.

## Revendications

1. Article absorbant de type culotte (1), comportant :
une partie côté abdomen (2) et une partie côté dos (3), et
des parties de scellage (5L, 5R) qui joignent des parties d'extrémité (2L, 2R) de la partie côté abdomen (2) dans une direction allant dans le sens de la largeur (W) et des parties d'extrémité (3L, 3R) de la partie côté dos (3) dans la direction allant dans le sens de la largeur (W), qui sont superposées de telle sorte que les surfaces intérieures (2i, 3i) de celles-ci sont orientées les unes vers les autres, les unes sur les autres, dans lequel
les parties de scellage (5L, 5R) comportent une pluralité de parties thermocollées (11) qui thermocollent les parties d'extrémité (2L, 2R) de la partie côté abdomen (2) dans la direction allant dans le sens de la largeur (W) et les parties d'extrémité (3L, 3R) de la partie côté dos (3) dans la direction allant dans le sens de la largeur (W) les unes sur les autres, les parties thermocollées (11) étant alignées dans une direction allant dans le sens de la longueur (L) tout en étant espacées les unes par rapport aux autres,
toutes les parties thermocollées (11) comportent chacune une partie mince (11bt) ayant une épaisseur relative de petite taille et une partie épaisse (11bT) ayant une épaisseur relative de grande taille, toutes les parties minces (11bt) étant agencées sur un côté intérieur des parties thermocollées (11) dans la direction allant dans le sens de la largeur (W),
les parties thermocollées (11) comportent chacune une rainure en retrait (11d), une paroi périphérique (11s) et une paroi inférieure (11b),
la paroi inférieure (11b) comporte la partie mince (11bt) ayant une épaisseur relative de petite taille et la partie épaisse (11bT) ayant une épaisseur relative de grande taille, et
l'épaisseur relative de petite taille et l'épaisseur relative de grande taille sont des dimensions dans une direction allant dans le sens de l'épaisseur (T) de l'article absorbant (1) .

2. Article absorbant de type culotte (1) selon la revendication 1, dans lequel
les parties de scellage (5L, 5R) joignent l'une des parties d'extrémité (2L) de la partie côté abdomen (2) dans la direction allant dans le sens de la largeur (W) et l'une des parties d'extrémité (3L) de la partie côté dos (3) dans la direction allant dans le sens de la largeur (W) l'une sur l'autre, et une autre partie d'extrémité (2R) de la partie côté abdomen (2) dans la direction allant dans le sens de la largeur (W) et une autre partie d'extrémité (3R) de la partie côté dos (3) dans la direction allant dans le sens de la largeur (W) l'une sur l'autre,
au moins deux parties thermocollées mutuellement adjacentes (11) des parties de scellage (5L) qui joignent lesdites unes parties d'extrémité (2L, 3L) de la partie côté abdomen (2) et de la partie côté dos (3) dans la direction allant dans le sens de la largeur (W) l'une sur l'autre comprennent chacune la partie mince (11bt) ayant une épaisseur relative de petite taille et la partie épaisse (11bT) ayant une épaisseur relative de grande taille, toutes les parties minces (11bt) étant agencées sur le côté intérieur (Wi) des parties thermocollées (11) dans la direction allant dans le sens de la largeur (W), et
au moins deux parties thermocollées mutuellement adjacentes (11) des parties de scellage (5R) qui joignent lesdites autres parties d'extrémité (2R, 3R) de la partie côté abdomen (2) et de la partie côté dos dans la direction allant dans le sens de la largeur (W) l'une sur l'autre comprennent chacune la partie mince (11bt) ayant une épaisseur relative de petite taille et la partie épaisse (11bT) ayant une épaisseur relative de grande taille, toutes les parties minces (11bt) étant agencées sur le côté intérieur des parties thermocollées (11) dans la direction allant dans le sens de la largeur (W).

3. Article absorbant de type culotte (1) selon la revendication 1 ou la revendication 2, dans lequel deux parties thermocollées mutuellement adjacentes (11) sur des parties d'extrémité supérieures (5LU, 5RU) des parties de scellage (5L, 5R) dans la direction allant dans le sens de la longueur (L) comprennent chacune la partie mince (11bt) ayant une épaisseur relative de petite taille et la partie épaisse (11bT) ayant une épaisseur relative de grande taille, toutes les parties minces (11bt) étant agencées sur le côté intérieur (Wi) de la partie thermocollée dans la direction allant dans le sens de la largeur (W) .

4. Article absorbant de type culotte (1) selon l'une quelconque des revendications 1 à 3, dans lequel deux parties thermocollées mutuellement adjacentes (11) sur des parties d'extrémité inférieures (5LB, 5RB) des parties de scellage (5L, 5R) dans la direction allant dans le sens de la longueur (L) comprennent chacune la partie mince (11bt) ayant une épaisseur relative de petite taille et la partie épaisse (11bT) ayant une épaisseur relative de grande taille, toutes les parties minces (11bt) étant agencées sur le côté intérieur des parties thermocollées (11) dans la direction allant dans le sens de la largeur (W).

5. Article absorbant de type culotte (1) selon l'une quelconque des revendications 1 à 4, dans lequel
l'une ou les deux parmi au moins les parties d'extrémité (2L, 2R) de la partie côté abdomen (2) dans la direction allant dans le sens de la largeur (W) et au moins les parties d'extrémité (3L, 3R) de la partie côté dos (3) dans la direction allant dans le sens de la largeur (W) comprennent une pluralité de parties en relief (13) mises en œuvre avec un motif prédéterminé, et
un écartement (P11) des parties thermocollées (11) dans la direction allant dans le sens de la longueur (L) est inférieur à un écartement (P13) des parties en relief (13) dans la direction allant dans le sens de la longueur (L).

6. Article absorbant de type culotte (1) selon l'une quelconque des revendications 1 à 5, dans lequel l'écartement (P11) des parties thermocollées (11) est inférieur à 1 mm.

7. Article absorbant de type culotte (1) selon l'une quelconque des revendications 1 à 6, dans lequel une dimension (L12) d'un intervalle (12) entre les parties thermocollées (11) dans la direction allant dans le sens de la longueur (L) est supérieure à une dimension (L11) des parties thermocollées (11) dans la direction allant dans le sens de la longueur (L).

8. Article absorbant de type culotte (1) selon l'une quelconque des revendications 1 à 7, dans lequel les rainures en retrait (11d) sont agencées du côté de la partie côté dos (3) et les parois inférieures (11b) sont agencées du côté de la partie côté abdomen (2).

9. Article absorbant de type culotte (1) selon l'une quelconque des revendications 1 à 7, dans lequel les rainures en retrait (11d) sont agencées du côté de la partie côté abdomen (2) et les parois inférieures (11b) sont agencées du côté de la partie côté dos (3).
